# EUROPEAN PATENT SPECIFICATION

(11) **EP 3 540 067 B1**
(45) Date of publication and mention of the grant of the patent: **30.04.2025**
(21) Application number: 17869759.5
(22) Date of filing: 14.11.2017
(51) Int. Cl.: C12N 5/0783, C12N 5/077, C12N 15/87

(54) **METHOD FOR DELIVERING EXOGENOUS MITOCHONDRIA INTO CELLS**
VERFAHREN ZUR EINFÜHRUNG EXOGENER MITOCHONDRIEN IN ZELLEN
MÉTHODE D'INTRODUCTION DE MITOCHONDRIES EXOGÈNES DANS DES CELLULES

(30) Priority: 14.11.2016 KR 20160151397
(43) Date of publication of application: 18.09.2019
(73) Proprietor: Paean Biotechnology Inc., Daejeon 34028 (KR)
(72) Inventor: CHOI, Yong-Soo, Gunpo-si Gyeonggi-do 15888 (KR); YUN, Chang-koo, Hwaseong-si Gyeonggi-do 18472 (KR); KIM, Mi-Jin, Namyangju-si Gyeonggi-do 12178 (KR); HWANG, Jung Uk, Sejong 30082 (KR); KIM, Chun-Hyung, Sejong-si 30130 (KR); LEE, Youngjun, Seoul 05348 (KR)
(74) Representative: Dehns
(86) International application number: PCT/KR2017/012882
(87) International publication number: WO 2018/088874

(56) References cited:
- WO-A1-2016/008937
- WO-A1-2016/008937
- WO-A1-2016/135723
- JP-A- 2002 218 843
- JP-A- 2014 097 068
- KR-A- 930 013 129
- US-A1- 2013 022 666
- US-A1- 2013 034 527
- ANDRES CAICEDO ET AL: "MitoCeption as a new tool to assess the effects of mesenchymal stem/stromal cell mitochondria on cancer cell metabolism and function", SCIENTIFIC REPORTS, vol. 5, 13 March 2015 (2015-03-13), pages 9073, XP055349992, DOI: 10.1038/srep09073
- T. KITANI ET AL: "Direct Human Mitochondrial Transfer: A Novel Concept Based on the Endosymbiotic Theory", TRANSPLANTATION PROCEEDINGS, vol. 46, no. 4, 1 May 2014 (2014-05-01), pages 1233 - 1236, XP055135789, ISSN: 0041-1345, DOI: 10.1016/j.transproceed.2013.11.133
- WU TING-HSIANG ET AL: "Mitochondrial Transfer by Photothermal Nanoblade Restores Metabolite Profile in Mammalian Cells", CELL METABOLISM, CELL PRESS, UNITED STATES, vol. 23, no. 5, 10 May 2016 (2016-05-10), pages 921 - 929, XP029536467, ISSN: 1550-4131, DOI: 10.1016/J.CMET.2016.04.007
- ANDRÉS CAICEDO ET AL: "Artificial Mitochondria Transfer: Current Challenges, Advances, and Future Applications", STEM CELLS INTERNATIONAL, vol. 2017, 1 January 2017 (2017-01-01), US, pages 1 - 23, XP055696104, ISSN: 1687-966X, DOI: 10.1155/2017/7610414
- MI JIN KIM ET AL: "Delivery of exogenous mitochondria via centrifugation enhances cellular metabolic function", SCIENTIFIC REPORTS, vol. 8, no. 1, 20 February 2018 (2018-02-20), XP055695889, DOI: 10.1038/s41598-018-21539-y
- CAICEDO, A. ET AL.: "MitoCeption as a New Tool to Assess the Effects of Mesenchymal Stem/Stromal Cell Mitochondria on Cancer Cell Metabolism and Function", SCIENTIFIC REPORTS, vol. 5, no. 9073, 2015, pages 1 - 10, XP055349992
- WU, T-H. ET AL.: "Mitochondrial Transfer by Photothermal Nanoblade Restores Metabolite Profile in Mammalian Cells", CELL METABOLISM, vol. 23, no. 5, 10 May 2016 (2016-05-10), pages 921 - 929, XP029536467
- KITANI, T. ET AL.: "Internalization of Isolated Functional Mitochondria: In volvement of Macropinocytosis", JOURNAL OF CELLULAR AND MOLECULAR MEDICINE, vol. 18, no. 8, 2014, pages 1694 - 1703, XP055135790
- CAICEDO, A. ET AL.: "Artificial Mitochondria Transfer: Current Challenges, Advances, and Future Applications", STEM CELLS INTERNATIONAL, vol. 2017, no. 7610414, 2 July 2017 (2017-07-02), pages 1 - 23, XP055500942

## Description

### Technical Field

The present invention relates to an *ex vivo* method for delivering exogenous mitochondria into recipient cells. More particularly, the present invention relates to a method for efficiently delivering mitochondria isolated from donor cells into the cytoplasm of recipient cells.

### Background Art

Mitochondrion is an organelle essential for survival of eukaryotic cells which is involved in the synthesis and regulation of adenosine triphosphate (ATP) as an energy source. The mitochondrion is associated with the control of various metabolic pathways *in vivo,* such as cell signal processing, cell differentiation, cell death, as well as cell cycle and cell growth.

Therefore, damage to mitochondria may lead to various diseases, and most known mitochondrial disorders are caused by inherited or acquired mutations occurring in mitochondrial DNA. For example, mitochondrial function may be impaired by swelling due to mitochondrial membrane potential abnormality, oxidative stress due to reactive oxygen species, free radicals, or the like, defects in oxidative phosphorylation function of mitochondria for energy production, and the like.

Specifically, mitochondrial dysfunction is known to be a direct or indirect cause of various diseases such as multiple sclerosis, encephalomyelitis, cerebral radiculitis, peripheral neuropathy, Reye's syndrome, Alper's syndrome, MELAS, migraine, psychosis, depression, seizure and dementia, apoplectic episode, optic atrophy, optic neuropathy, retinitis pigmentosa, cataract, hyperaldosteronism, hypoparathyroidism, myopathy, muscle atrophy, myoglobinuria, muscle tension inhibition, muscle pain, deteriorated exercise tolerance, renal tubulopathy, kidney failure, liver failure, liver dysfunction, hepatomegaly, iron red blood cell anemia, neutropenia, thrombocytopenia, diarrhea, villous atrophy, multiple vomiting, dysphagia, constipation, sensorineural hearing loss, epilepsy, mental retardation, Alzheimer's disease, Parkinson's disease, and Huntington's disease.

In order to treat such mitochondria-related diseases, research on materials that can be used for protecting mitochondria or restoring mitochondrial dysfunction, methods that allow efficient targeted delivery of such materials, or the like have continued. However, from the viewpoint that there are problems of a rather limited therapeutic range, occurrence of side effects, or the like, there has not yet been developed an epoch-making treatment for mitochondria-related diseases.

WO 2016/008937 relates to methods for the intercellular transfer of isolated mitochondria in recipient cells. Andres Caicedo et al. (SCIENTIFIC REPORTS, vol. 5, page 9073) relates to MitoCeption as a new tool to assess the effects of mesenchymal stem/stromal cell mitochondria on cancer cell metabolism and function. WO 2016/135723 relates to human bone-marrow cells enriched with functional mitochondria, methods for their production, and therapeutic methods utilizing such cells. Kitani et al. (TRANSPLANTATION PROCEEDINGS, vol. 46, no. 4, pages 1233 - 1236) relates to direct human mitochondrial transfer: A novel concept based on the endosymbiotic theory. Wu Ting-Hsiang et al. (CELL METABOLISM, CELL PRESS, US, vol. 23, no. 5, pages 921 - 929) discloses that mitochondrial transfer by photothermal nanoblade restores metabolite profiles in mammalian cells.

In addition, as a method for fundamentally blocking mitochondria-related diseases, a technique for extracting a nuclear genetic material from an oocyte having mitochondrial mutations and transplanting the nuclear genetic material into an oocyte having normal mitochondria is emerging. However, biological controversy and ethical controversy have prevented relevant laws from being passed.

Accordingly, in the course of making an effort to find a method that effectively enhances intracellular mitochondrial activity while not conflicting with bioethics, the present inventors have devised a technique capable of injecting healthy mitochondria without causing cell damage, and thus have completed the present invention.

### Technical Problem

An object of the present invention is to provide a method for injecting mitochondria isolated from cells into specific cells. In addition, another object of the present invention is to provide cells having improved mitochondrial function, the cells being obtained by delivering normal mitochondria into cells at a high delivery rate without damaging the cells to which the mitochondria are to be administered.

### Solution to Problem

In order to achieve the above objects, according to an aspect of the present invention, there is provided an *ex vivo* method for delivering exogenous mitochondria into recipient cells, comprising a) a step of mixing the recipient cells with mitochondria isolated from donor cells, and b) a step of centrifuging the resulting mixture, the method further comprising a step of adding a surfactant before the step b), wherein the donor cells and the recipient cells are respectively any one selected from the group consisting of somatic cells, stem cells, cancer cells, and combinations thereof.

### Advantageous Effects of Invention

Through the method of the present invention, mitochondria isolated from normal cells can be effectively injected into recipient cells. In particular, the method of the present invention has advantages of exhibiting a very high injection yield and causing no cell damage, since it is possible to regulate an amount of mitochondria to be delivered into cells.

### Brief Description of Drawings

Figures 19, 20 and 22 relate to methods in accordance with the invention.
FIG. 1 illustrates results obtained by observing intracellular mitochondria with photofluorography.
FIG. 2 illustrates results obtained by observing isolated mitochondria with photofluorography.
FIG. 3 illustrates a schematic diagram for a mitochondrial isolation process.
FIG. 4 illustrates a graph obtained by comparing mitochondrial activity depending on a donor cell type.
FIG. 5 illustrates a graph obtained by identifying mitochondrial function by measuring an ATP content in isolated mitochondria.
FIG. 6 illustrates a result obtained by identifying high purity of isolated mitochondria by analyzing expression of mitochondrial markers and nuclear markers.
FIGS. 7A to 7C illustrate results obtained by identifying mitochondrial delivery into UC-MSCs using a mitochondrial staining pattern.
FIGS. 7D to 7F illustrate results obtained by identifying mitochondrial delivery with 3D Z-stack.
FIGS 7G to 7I illustrate results obtained by identifying a survival rate of isolated mitochondria using mitochondria-specific MitoTracker probes.
FIG. 8 illustrates results obtained by identifying a delivery rate of mitochondria, which have been delivered into UC-MSCs, through FACS analysis.
FIG. 9 illustrates a result obtained by identifying, using qPCR, delivery of UC-MSC-derived mitochondria into recipient cells (L6 cells).
FIG. 10 illustrates a result obtained by identifying, using real-time PCR, that mitochondrial delivery is effectively done through centrifugation.
FIG. 11 illustrates results obtained by identifying, through FACS analysis, mitochondrial delivery into various cells.
FIG. 12 illustrates a process in which donor cell-derived mitochondria are delivered into recipient cells and then the resulting recipient cells are used.
FIG. 13 illustrates results obtained by analyzing, through FACS, a delivery rate of exogenous mitochondria to be mixed depending on a content thereof.
FIG. 14 illustrates a graph obtained by analyzing, through FACS, a delivery rate of exogenous mitochondria to be mixed depending on a content thereof.
FIG. 15 illustrates an amount of exogenous mitochondria delivered into recipient cells depending on a concentration of mitochondria to be mixed.
FIG. 16 illustrates a photograph obtained by taking, with a confocal scanning microscope, exogenous mitochondria delivered into recipient cells.
FIG. 17 illustrates results obtained by identifying, through FACS analysis, an optimal condition for mitochondrial delivery.
FIGS. 18A and 18B illustrate results obtained by delivering mitochondria under the optimal condition for mitochondrial delivery and then identifying the delivered mitochondria with a confocal microscope.
FIG. 19 illustrates results obtained by analyzing, through FACS, a mitochondrial delivery rate depending on an incubation time after addition of a surfactant.
FIG. 20 schematically illustrates, as a bar graph, results obtained by analyzing, through FACS, a mitochondrial delivery rate depending on an incubation time after addition of a surfactant.
FIG. 21 illustrates results obtained by measuring a mitochondrial delivery rate obtained through a process of adding a surfactant to recipient cells and performing incubation without a centrifuging process.
FIG. 22 illustrates results obtained by measuring a mitochondrial delivery rate in a case where recipient cells are treated with a surfactant at various concentrations and treatment times, and then mitochondria are delivered into the recipient cells through centrifugation.

### Detailed Description of Invention

Hereinafter, the present invention will be described in detail.

In an aspect of the present invention, there is provided an *ex vivo* method for delivering exogenous mitochondria into recipient cells, comprising a) a step of mixing the recipient cells with mitochondria isolated from donor cells, and b) a step of centrifuging the resulting mixture, the method further comprising a step of adding a surfactant before step b), wherein the donor cells and the recipient cells are respectively any one selected from the group consisting of somatic cells, stem cells, cancer cells, and combinations thereof.

First, a step of mixing recipient cells with mitochondria isolated from donor cells is provided.

Here, "donor cells" refer to cells that provide mitochondria. In addition, the donor cells may be normal cells which contain normal mitochondria. Specifically, the donor cells may be cells isolated from a mammal including an animal or a human. The donor cells are selected from the group consisting of somatic cells, stem cells, cancer cells and combinations thereof.

In addition, the somatic cells may be any one selected from the group consisting of muscle cells, hepatocytes, fibroblasts, epithelial cells, neurons, adipocytes, osteocytes, leukocytes, lymphocytes, mucosal cells, and combinations thereof; may be any one selected from the group consisting of muscle cells, hepatocytes, fibroblasts, and combinations thereof; or may be muscle cells or hepatocytes. Preferably, the donor cells may be muscle cells with excellent mitochondrial activity.

In addition, the stem cells refer to undifferentiated cells having an ability to differentiate into various types of tissue cells. The stem cells may be any type of stem cells such as adult stem cells, mesenchymal stem cells, induced pluripotent stem cells, and tissue-derived stem cells.

In addition, the cells may be any cancer cells selected from the group consisting of squamous cell carcinoma, small cell lung cancer, non-small cell lung cancer, lung cancer, adenocarcinoma, peritoneal cancer, colon cancer, biliary tumor, nasopharyngeal cancer, laryngeal cancer, bronchial cancer, oral cancer, osteosarcoma, gallbladder cancer, kidney cancer, leukemia, bladder cancer, melanoma, brain cancer, glioma, brain tumor, skin cancer, pancreatic cancer, breast cancer, liver cancer, bone marrow tumor, esophageal cancer, colorectal cancer, stomach cancer, cervical cancer, prostate cancer, ovarian cancer, head and neck cancer, and rectal cancer cells.

On the other hand, "recipient cells" refer to cells into which mitochondria are introduced. In addition, the recipient cells may be normal cells, and may be any one of white blood cells and lymphocytes which are immune-related cells. In addition, the recipient cells may be abnormal cells that contain abnormal mitochondria due to a congenital or acquired factor. The recipient cells are cells selected from the group consisting of somatic cells, stem cells, cancer cells and combinations thereof.

In addition, the donor cells and the recipient cells may be allogeneic or xenogeneic to each other. The donor cells and the recipient cells may be obtained from the same subject or from different subjects.

For step a), a method of isolating mitochondria from the donor cells can be selected from various known methods, for example, a method of isolating mitochondria using a specific buffer solution or a method of extracting mitochondria using potential difference and magnetic field.

For the method of isolating mitochondria, in order to maintain mitochondrial activity, mitochondria may be obtained by disrupting a cell and performing centrifugation. Isolation of mitochondria may be done by a step of culturing donor cells and performing a primary centrifugation of a composition that contains such cells, to produce a pellet, a step of resuspending the pellet in a buffer solution and performing homogenization, a step of performing a secondary centrifugation of the homogenized solution, to prepare supernatant, and a step of performing a tertiary centrifugation of the supernatant, to purify mitochondria. Here, from the viewpoint of maintaining cell activity, it is preferable that the time for performing the secondary centrifugation is regulated to be shorter than the time for performing the primary and tertiary centrifugations. It is possible to increase the speed as the step proceeds from the primary centrifugation to the tertiary centrifugation.

Specifically, the primary to tertiary centrifugations may be performed at a temperature of 0°C to 40°C, and preferably at a temperature of 3°C to 5°C. In addition, the centrifugations may be performed for 0.5 minutes to 50 minutes, 1 minute to 20 minutes, or 2 minutes to 30 minutes, and preferably 5 minutes. In addition, the time for performing the centrifugation can be appropriately adjusted depending on the number of centrifugations, a content of a sample, and the like. The centrifugations may be performed with a speed of 1 × g to 2,400 × g, 350 × g to 2,000 × g, or 600 × g to 1,600 × g, and preferably 1,500 × g.

In addition, the primary centrifugation may be performed with a speed of 100 to 1,000 × g, 200 to 700 × g, or 300 to 450 × g. In addition, the secondary centrifugation may be performed with a speed of 1 to 2,000 × g, 25 to 1,800 × g, or 500 to 1,600 × g. In addition, the tertiary centrifugation may be performed with a speed of 100 to 20,000 × g, 500 to 18,000 × g, or 800 to 15,000 × g.

Next, a step of delivering exogenous mitochondria into the recipient cells is provided, the step comprising a step of centrifuging the resulting mixture.

Here, the centrifugation may be performed at 100 × g, 300 × g, 500 × g, 800 × g, 1,000 × g, 1,200 × g, 1,500 × g, 1,800 × g, 2,000 × g, 2,400 × g, 3,000 × g, 5,000 × g, or 10,000 × g. In addition, the time for performing the centrifugation may be 0.1 minutes to 60 minutes, but is not limited thereto. Specifically, the time may be 1 minute, 2 minutes, 3 minutes, 5 minutes, 10 minutes, 20 minutes, or 30 minutes.

The centrifugation may be performed at a temperature of 0°C to 40°C, 20°C to 38°C, or 30°C to 37°C.

As such, by applying a centrifugal force to both the recipient cells and the mitochondria isolated from the donor cells, the mitochondria can be delivered into the recipient cells with high efficiency while causing less damage to the recipient cells. In addition, in a case where the recipient cells have mitochondria with defects, mitochondria isolated from donor cells that contain normal mitochondria can be injected into the recipient cells, so that functions of the recipient cells due to mitochondrial defects can be restored.

In addition, in the step b), it may be more effective to perform the centrifugation of the mixture in a test tube whose diameter gradually narrows toward the bottom, in terms of a centrifugal force to be applied to the recipient cells and mitochondria, and contact efficiency therebetween. The centrifugation may be performed one to three additional times under the same condition.

In an embodiment of the present invention, in a case where the centrifugation is performed with a speed of 1,500 × g for 5 minutes, it was found that mitochondria are best delivered into the recipient cells.

In addition, in the step a), the mitochondria may be mixed, at varying amounts, with the recipient cells. Specifically, the mitochondria may be contained in an amount of 0.005 to 25 µg, 0.01 to 23 µg, or 0.1 to 20 µg, per 1 × 10⁵ recipient cells. Specifically, the mitochondria may be contained in an amount of 0.1 µg, 1 µg, 3 µg, 5 µg, 10 µg, 15 µg, or 18 µg, per 1 × 10⁵ recipient cells. In a case where the mitochondria are contained in an amount of fewer than 0.005 µg per 1 × 10⁵ recipient cells, a somewhat decreased delivery yield may be obtained.

Before performing the step b), the method further comprises a step of adding a surfactant to the mixture obtained in the step a). The surfactant is used to enhance cell membrane permeability of the recipient cells. A time point at which the surfactant is added may be before, during, or after mixing of the recipient cells with the mitochondria. In addition, after the surfactant is added to the recipient cells, the recipient cells may be incubated for a certain period of time in order to increase the cell membrane permeability of the recipient cells. The time for which the recipient cells are left to stand may be 0.1 to 60 minutes. Specifically, the time may be 1 minute, 5 minutes, 10 minutes, 20 minutes, or 30 minutes, but is not limited thereto.

Specifically, the surfactant is preferably a nonionic surfactant, and may be a poloxamer. Here, the poloxamer is a triblock copolymer composed of a central hydrophobic chain of polyoxypropylene flanked by two hydrophilic chains of polyoxyethylene. In addition, the surfactant in the mixture may have a concentration of 1 to 100 mg/ml, 3 to 80 mg/ml, or 5 to 40 mg/ml, and preferably 10 to 30 mg/ml.

In addition, before performing the step b), the method may further comprise a step of incubating the mixture obtained in the step a) under a predetermined time and temperature condition. The incubation may be performed at a temperature of 0°C to 40°C, 20°C to 38°C, or 30°C to 37°C. In addition, the incubation may be performed for 0.1 to 4 hours, 0.5 to 3.8 hours, or 0.8 to 3.5 hours. In addition, the incubation may be performed for a predetermined time, after step b) is performed so as to deliver the mitochondria to the recipient cells. In addition, the incubation time can be appropriately selected depending on a cell type and an amount of mitochondria.

In addition, before performing the step b), the method may further comprise a step of adding a surfactant to the mixture obtained in the step a) and performing incubation. Here, the surfactant and a condition for the incubation are the same as those described above.

The delivery method according to the present invention can improve mitochondrial function of a cell into which an exogenous mitochondrion is to be introduced, and the cell thus obtained can be used to improve mitochondrial function and to treat or prevent a mitochondrial dysfunction disease.

Hereinafter, preferred examples of the present invention will be described in order to facilitate understanding of the present invention. However, the following examples are provided only for the purpose of easier understanding of the present invention, and the present invention is not limited by the following examples.

### Preparation Example 1. Preparation of cells

(This Example is not part of the invention.) Human adipocyte-derived mesenchymal stem cells (AD-MSCs; PCS-500-011, passage #7), human breast cancer cell line (MCF-7; HTB-22, passage #20), human adenocarcinoma alveolar epithelial cells (A549; CCL-185, passage #20), human leukemia cell line (K562; CCL-243, passage #20), human liver cell line (WRL-68; CL-48, passage #9), human dermal fibroblasts (HDFs; PCS-201-012, passage #9), and human natural killer cell line (NK-92; CRL-2407, passage #10) were purchased from the Animal Type Culture Collection (Manassas, VA). Human bone marrow-derived mesenchymal stem cells (BM-MSCs; MSC-001F, passage #7) were purchased from STEMCELL Technologies Inc. (Vancouver, Canada). In order to compare delivery efficiency (%) depending on a cell type, all cell lines were kept in a 37°C bath until complete thawing is achieved. Thereafter, the cell lines were used immediately for mitochondrial delivery.

UC-MSCs were cultured in α-MEM medium (Hyclone Laboratories Inc., Logan, UT) supplemented with 10% fetal bovine serum (FBS; Hyclone Laboratories Inc.), 100 µg/ml of streptomycin and 100 IU/ml of penicillin (P/S; Hyclone Laboratories Inc.), and 10 ng/ml of basic fibroblast growth factor (CHA Meditech Co., Ltd., Daejeon, Korea). When cell confluency reached 80% to 90%, the UC-MSCs at the stage of passage #5 to # 7 were used. For reference, the present experiment was conducted under the approval of the Institutional Audit Committee at CHA University (Seongnam, Korea; IRB No. 201412-BR-003-02).

The rat skeletal muscle-derived cell line, L6 (CRL-1458), was purchased from the Animal Type Culture Collection (Manassas, VA). The cells were cultured in DMEM medium (DMEM; Hyclone Laboratories Inc.) supplemented with 10% FBS and 1% P/S, and the L6 cells at the stage of passage #6 to #10 were used.

### Preparation Example 2. Fluorescence image analysis

(This Example is not part of the invention.) In the following example, a TCS SP5 II confocal microscope (Leica, Heidelberg, Germany) with 10× and 20× numerical apertures was used to track mitochondrial delivery. Digital images were identified using a 2.6 version of the Leica LAS AF software (Leica Microsystems, Mannheim, Germany).

### Example 1. Mitochondrial isolation (This Example is not part of the invention.)

### 1.1. Isolation of mitochondria in L6 cells

L6 cultured in the Preparation Example 2 was stained with a greenish mitochondria-specific marker (Mitotracker green). An observation was made using a fluorescence microscope to identify whether green fluorescence was well labeled in the intracellular mitochondria.

In order to extract mitochondria, a hemocytometer was used to measure the number of cells, and cells in an amount of about 2 × 10⁷ cells/ml were collected. Thereafter, the cell line was subjected to primary centrifugation at a temperature of about 4°C for 10 minutes with a speed of 350 × g. Here, the resulting pellet was collected, and resuspended and homogenized, using a disposable 1 ml syringe, in a SHE buffer solution (0.25 M sucrose, 20 mM HEPES (pH 7.4), 2 mM EGTA, 10 mM KCl, 1.5 mM MgCl₂ and 0.1% BSA (defatted bovine serum albumin)) supplemented with a protease inhibitor (Roche Diagnostics, Mannheim, Germany). A composition containing the pellet was subjected to secondary centrifugation at a temperature of about 4°C for 3 minutes with a speed of 1,100 × g, to obtain supernatant. Then, the supernatant was subjected to tertiary centrifugation at about 4 °C for 15 minutes with a speed of 12,000 × g, to isolate mitochondria from the cell line.

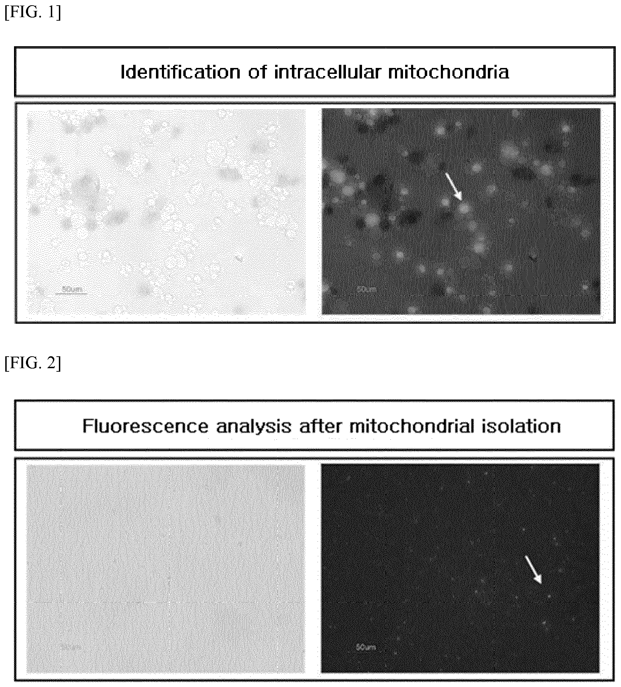

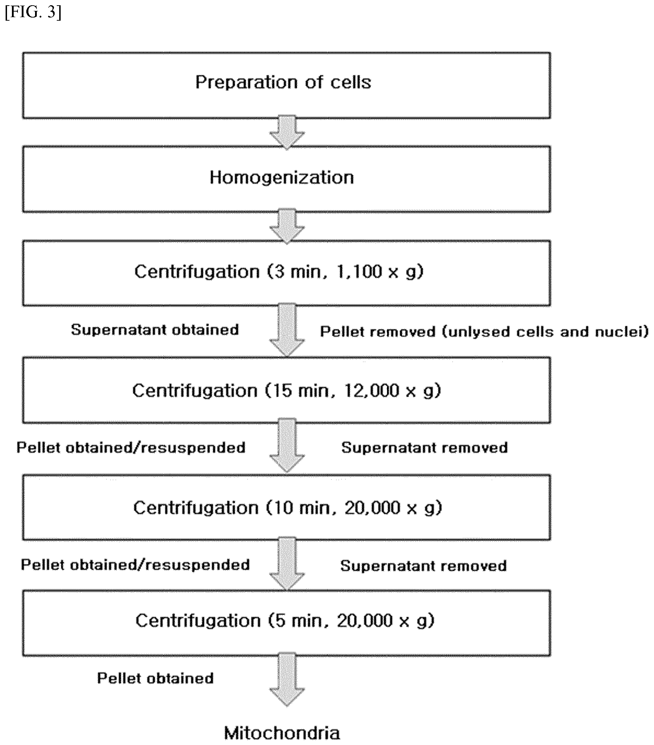

In order to identify the isolated mitochondria, the intracellular mitochondria labeled with green fluorescence before performing the isolation were taken with a normal microscope and a fluorescence microscope, and the results are illustrated in FIG. 1; and the isolated mitochondria were taken with a normal microscope and a fluorescence microscope, and the results are illustrated in FIG. 2. A flow chart for the mitochondrial isolation experiment is illustrated in FIG. 3.

As illustrated in FIGS. 1 and 2, it was identified that the mitochondria present in the L6 cells before isolation had been isolated.

### 1.2. Isolation of mitochondria in human umbilical cord-derived mesenchymal stem cells

Fractional centrifugation was used to isolate mitochondria from human umbilical cord-derived mesenchymal stem cells (UC-MSCs, provided by CHA bundang medical center, IRB No. 201412-BR-003-02). In order to extract mitochondria, a hemocytometer was used to measure the number of cells, and cells in an amount of about 2 × 10⁷ cells were collected.

The cell line was subjected to primary centrifugation at a temperature of about 4°C for 10 minutes with a speed of 350 × g. Here, the resulting pellet was collected, and resuspended and homogenized, using a disposable 1 ml syringe, in a SHE buffer solution (0.25 M sucrose, 20 mM HEPES (pH 7.4), 2 mM EGTA, 10 mM KCl, 1.5 mM MgCl₂ and 0.1% BSA (defatted bovine serum albumin)) supplemented with a protease inhibitor (Roche Diagnostics, Mannheim, Germany).

A composition containing the pellet was subjected to secondary centrifugation at a temperature of about 4°C for 3 minutes with a speed of 1,100 × g, to remove unlysed cells and cellular debris, and supernatant was obtained. Then, in order to pelletize the mitochondria, the supernatant was subjected to tertiary centrifugation at a temperature of about 4°C for 15 minutes with a speed of 12,000 × g. The mitochondrial pellet was resuspended in 500 µl of the SHE buffer and subjected to centrifugation at about 4°C for 5 minutes with a speed of 20,000 × g. After removal of supernatant, the pellet was resuspended in 50 µl of PBS and stored on ice until measurement was performed. All analyses were performed with freshly isolated mitochondria.

### Example 2: Identification of activity and function of mitochondria isolated from donor cells (This Example is not part of the invention.)

### 2.1. Measurement of activity of mitochondria isolated from donor cells

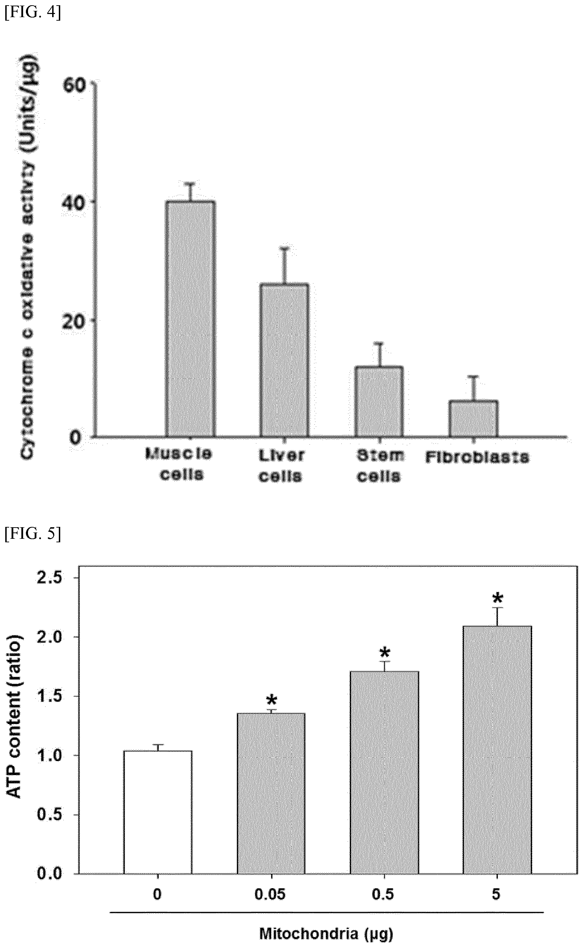

Mitochondria were further isolated from the human liver cell line, WRL-68, the human dermal fibroblasts, HDFs, and the human umbilical cord-derived mesenchymal stem cells, UC-MSCs, according to the method of Example 1. In order to measure activity of the mitochondria thus isolated, a cytochrome c oxidative activity assay kit (BioVision Inc.) was used. Mitochondria in each cell were allowed to react with a reduced form of cytochrome c contained in the kit, and absorbance was measured at 550 nm for 30 minutes at 1-minute intervals. Oxidative capacity of mitochondria for the reduced form was calculated from changes in absorbance measured over time. Cytochrome c oxidative activity of the mitochondria obtained from each cell line was measured and is illustrated in FIG. 4.

As illustrated in FIG. 4, ATP activity capacity of the isolated mitochondria appeared to be in the order of muscle cells, hepatocytes, umbilical cord-derived stem cells, and fibroblasts. From this, it can be seen that different mitochondrial activity is exhibited depending on a tissue.

### 2.2. Identification of function of mitochondria isolated from donor cells

In order to identify the function of mitochondria isolated from the UC-MSCs, for the isolated mitochondria, a bicinchoninic acid (BCA) assay was used to quantify a concentration of mitochondrial protein, and ATP was measured at various concentrations (0.05 µg, 0.5 µg, and 5 µg). An amount of ATP in the mitochondria was measured using the CellTiter-Glo luminescence kit (Promega Corporation, Madison, WI) which generates a luminescence signal in proportion to the amount of ATP.

The mitochondria at each concentration were mixed with 50 µl of PBS and then prepared in a 96-well plate. 50 µl of a test solution contained in the kit was equally added thereto, and allowed to react at room temperature for 30 minutes. Then, the amount of ATP was measured by measuring a luminescence signal using a luminescence microplate reader. This result is illustrated in FIG. 5.

As illustrated in FIG. 5, the amount of ATP was increased dependently on and in proportion to the concentration of mitochondria, from which it was possible to identify the function of mitochondria.

### Example 3. Measurement of purity of mitochondria isolated from donor cells

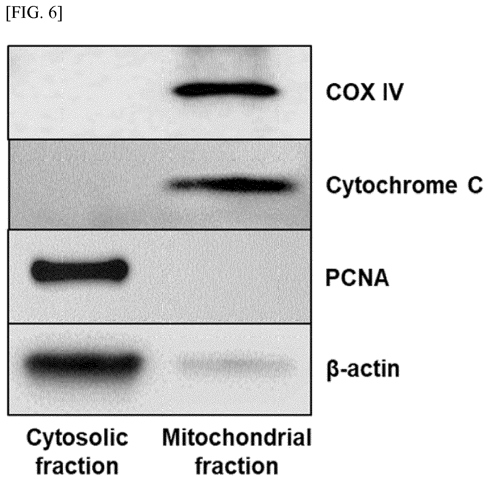

(This Example is not part of the invention.) In order to measure purity of mitochondria isolated from the UC-MSCs, expression of mitochondrial markers [cytochrome C oxidase (COX IV) and cytochrome c] and nuclear markers [proliferating cell nuclear antigen (PCNA) and β-actin] in a cytosolic fraction and a mitochondrial fraction which had been obtained in the mitochondrial isolation process was identified by Western blot analysis. This result is illustrated in FIG. 6.

As illustrated in FIG. 6, it was identified that COX IV and cytochrome c are expressed in the isolated mitochondrial fraction, while PCNA and β-actin are not expressed therein. From this, it was identified that the isolated mitochondria have high purity.

### Example 4. Identification of delivery of mitochondria isolated from donor cells (This Example is not part of the invention.)

### 4.1. Identification of delivery and activity of mitochondria isolated from WRL-68

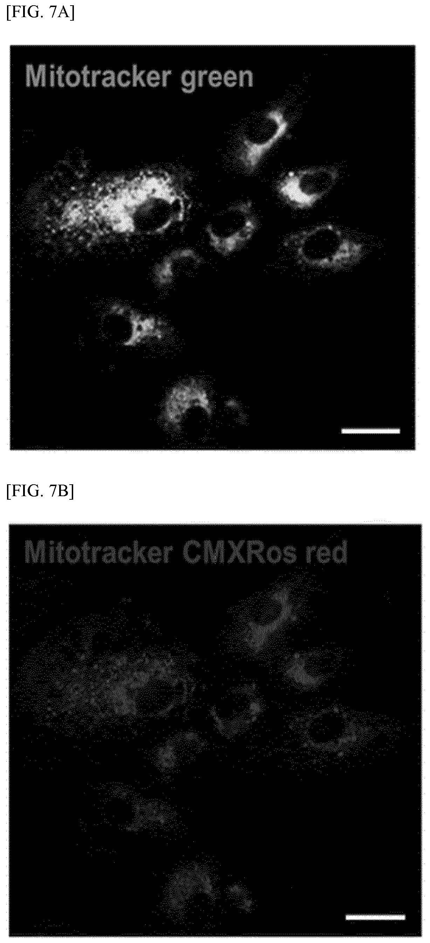

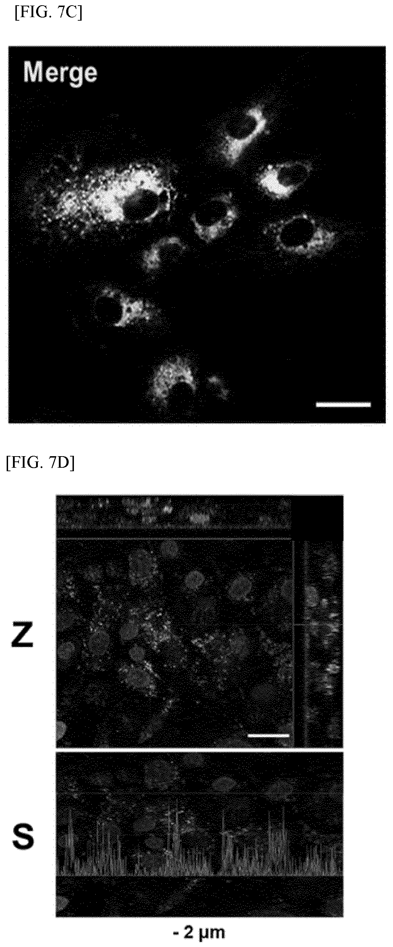

Mitochondria-specific MitoTracker probes were used to identify delivery of the mitochondria isolated from the WRL-68 and activity of the delivered mitochondria. Viability and respiratory ability of the mitochondria were identified using the feature that a MitoTracker Red CMXRos (CMXRos) probe with red fluorescence accumulates dependently on mitochondrial membrane potential (MMP). In addition, mitochondria in UC-MSCs which are recipient cells were stained with a MitoTracker Green (MTG) probe. Exogenous mitochondria isolated from WRL-68 stained with a MitoTracker Red CMXRos probe were mixed with the UC-MSCs stained with the MitoTracker Green probe. Then, centrifugation proceeded. Images obtained by making an observation with each of the MitoTracker Red CMXRos (CMXRos) probe and the MitoTracker Green (MTG) probe are illustrated in FIGS. 7A and 7B, respectively, and a merged image of the two images is illustrated in FIG. 7C.

As illustrated in FIGS. 7A to 7C, the exogenous mitochondria (red fluorescence) which had been delivered appeared as an image (yellow fluorescence) merged with the mitochondria (green fluorescence) inherent in the UC-MSCs. From this, it was found that intact mitochondria can be obtained by the mitochondrial isolation method of the present invention.

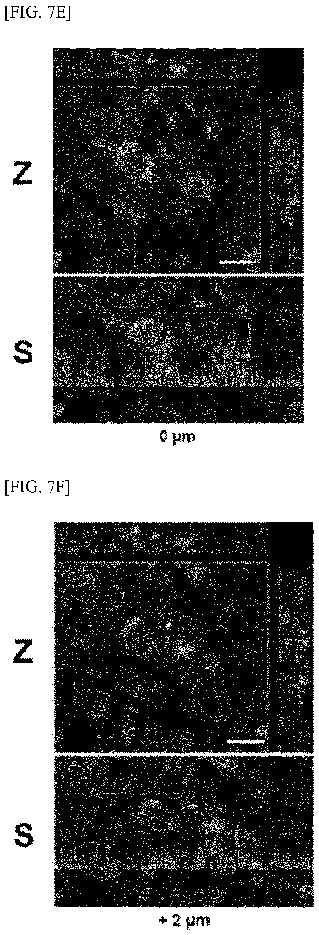

In addition, 3-dimensional z-stack images of the UC-MSCs having the exogenous mitochondria stained with the MitoTracker Red CMXRos (CMXRos) probe were observed. These results are illustrated in FIGS. 7D to 7F. On the basis of FIG. 7E, FIG. 7D is an image of a portion which is located lower by 2 µm, and FIG. 7F is an image of a portion which is located higher by 2 µm.

As illustrated in FIGS. 7E to 7F, the mitochondria delivered into the UC-MSCs, which are recipient cells, were identified through yellow fluorescence that occurs in a case of being merged. From this, it was found that the mitochondria are effectively delivered into the recipient cells through centrifugation. In addition, it was found that activity of the delivered mitochondria is maintained.

### 4.2. Identification of delivery and activity of mitochondria isolated from UC-MSCs

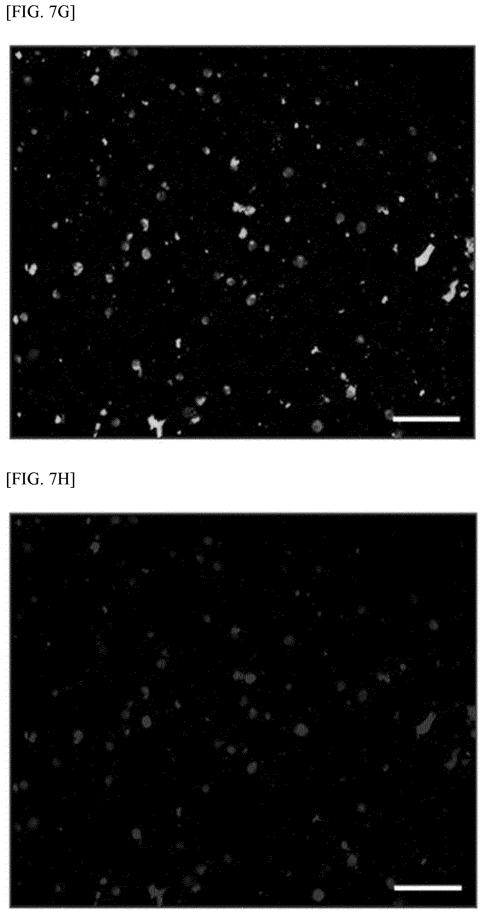

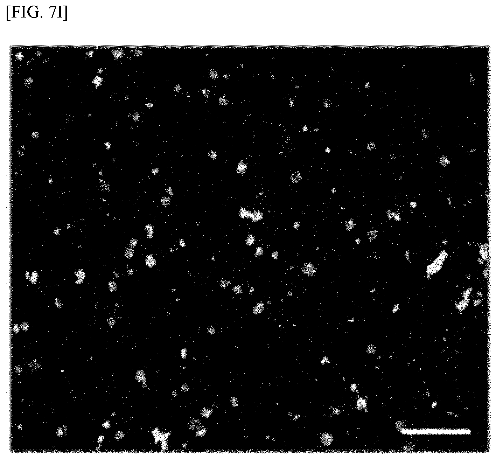

The mitochondrial-specific MitoTracker Red CMXRos (CMXRos) probe was used to identify delivery of the mitochondria isolated from the UC-MSCs and activity of the delivered mitochondria. In addition, intracellular mitochondria were stained with the MitoTracker Green (MTG) probe. In addition, the UC-MSCs were stained with a MitoTracker probe before mitochondrial isolation and incubated at 37°C for 30 minutes. After undergoing the mitochondrial isolation process, the mitochondrial pellet was mixed with 10 µl of PBS, placed on a slide, covered with a cover slide, and observed with a fluorescence microscope. Images obtained by making an observation with each of the MitoTracker Red CMXRos (CMXRos) probe and the MitoTracker Green (MTG) probe are illustrated in FIGS. 7G and 7H, respectively, and a merged image of the two images is illustrated in FIG. 7I.

From FIG. 7G to FIG. 7I, it was found that intact mitochondria can be obtained by the mitochondrial isolation method of the present invention.

### 4.3. Identification of mitochondrial delivery using FACS analysis

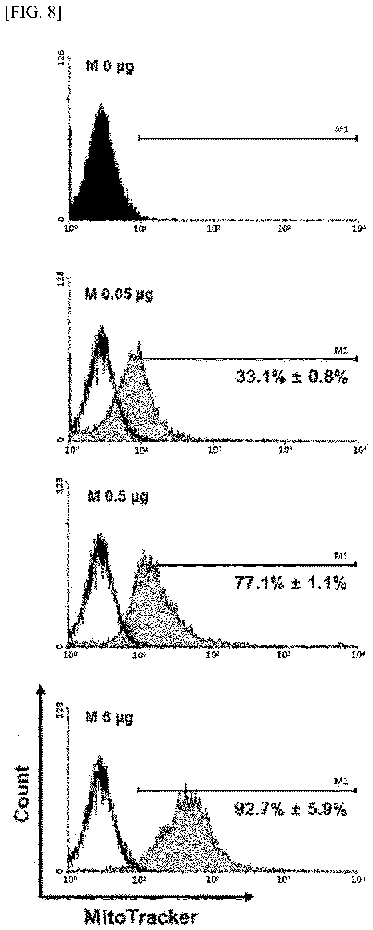

In order to identify a mitochondrial delivery rate, FACS was performed in the same manner as in Example 5.1 using MitoTracker Green. These results are illustrated in FIG. 8.

As illustrated in FIG. 8, delivery rates which appear in green fluorescence in proportion to amounts (0.05, 0.5, and 5 µg) of mitochondria delivered into UC-MSCs were increased to 33.1 ± 0.8%, 77.1 ± 1.1% and 92.7 ± 5.9%, respectively. From this, it was found that a delivery rate is increased in proportion to an amount (concentration (µg) of mitochondrial protein) of mitochondria delivered into the recipient cells.

### 4.4. Identification of mitochondrial delivery through qPCR analysis

In order to demonstrate mitochondrial delivery, donor cells or recipient cells after mitochondrial delivery were subjected to conventional PCR using specific primers, to demonstrate mtDNA expression in the recipient cells.

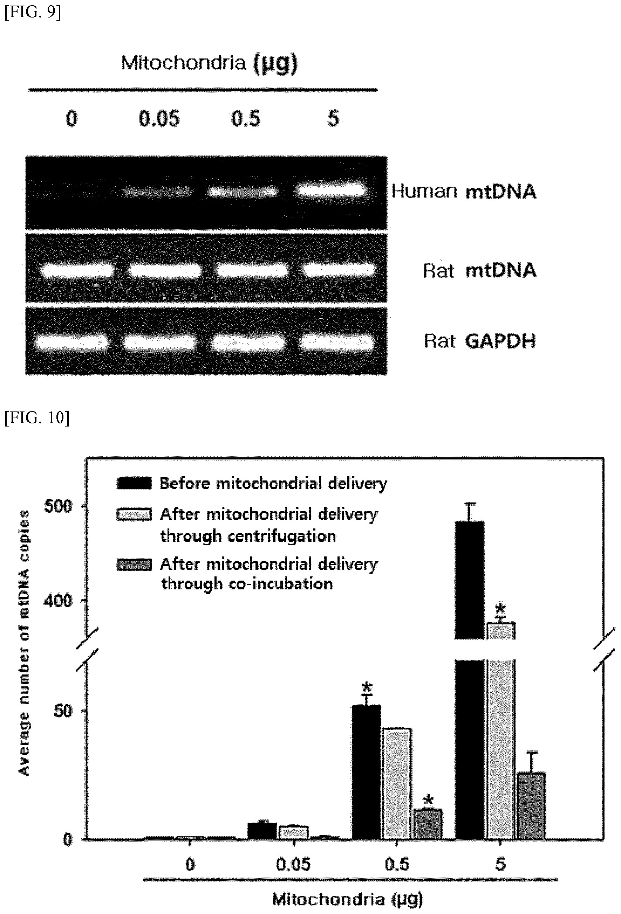

Mitochondria extracted from the human umbilical cord-derived mesenchymal stem cells, UC-MSCs, as donor cells were delivered into rat L6 cells, and then a quantitative polymerase chain reaction (qPCR) assay was used to identify expression of human-mtDNA, rat-mtDNA, and rat-GAPDH. Here, specific primers for human UC-MSCs and rat L6 cells were used. This result is illustrated in FIG. 9.

As illustrated in FIG. 9, expression of human-mtDNA in rat cells was increased dependently on an amount of mitochondria delivered into L6 cells. On the other hand, there was no change in expression of rat-mtDNA. From this, it was found that exogenous mitochondria have been delivered into the recipient cells into which mitochondria were to be delivered.

### 4.5. Identification of mitochondrial delivery through real-time PCR analysis

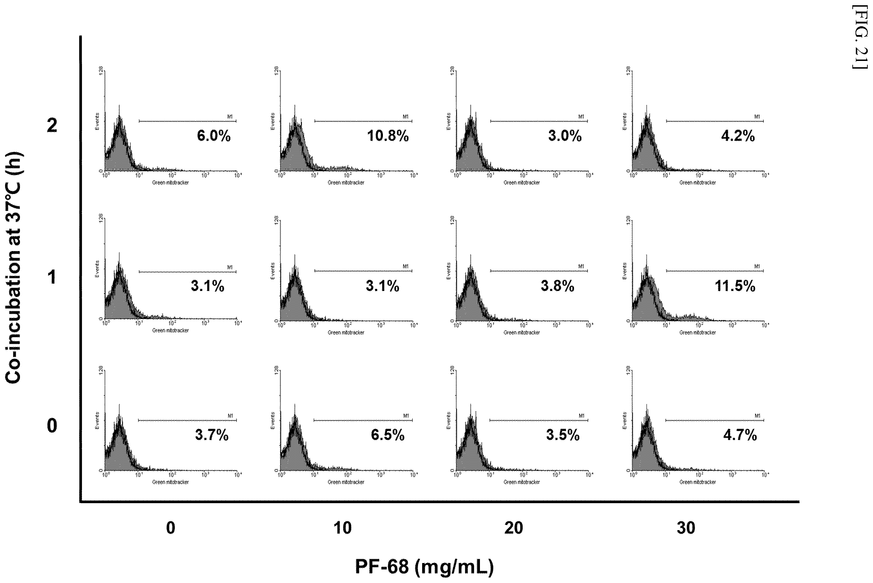

In order to obtain quantitative results together with the qualitative results identified in Example 4.4, a comparison was made between the number of human-mtDNA copies specific for the delivered mitochondria and the number of human-mtDNA copies before mitochondrial delivery, in the L6 cells into which exogenous mitochondria had been delivered. This result is illustrated in FIG. 10. In FIG. 21, the black bar (first from the top) represents the result obtained by identifying the number of mtDNA copies for a concentration of mitochondria to be delivered after extracting the mitochondria. The light gray bar (second from the top) represents the result obtained by identifying the number of mtDNA copies in recipient cells after mitochondrial delivery into the recipient cells through centrifugation. The dark gray bar (third from the top) represents the result obtained by identifying the number of mtDNA copies after mitochondrial delivery through co-incubation (24 hours) rather than centrifugation, which is intended to show a comparative numerical value relative to a delivery rate obtained through a centrifugation method.

As illustrated in FIG. 10, a mitochondrial delivery rate obtained through centrifugation appeared to be about 80% to 85%. In addition, a mitochondrial delivery rate obtained through the co-incubation appeared to be a numerical value which is as remarkably low as less than 10%. From this, it was found that the mitochondrial delivery method through centrifugation is effective.

### 4.6. Identification of mitochondrial delivery into various cells

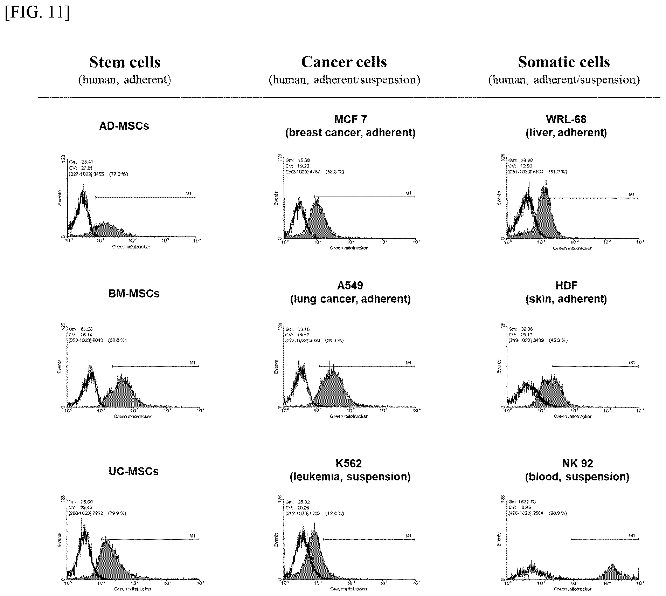

In order to compare differences in mitochondrial delivery depending on the nature of recipient cells, a delivery rate of mitochondria into various cells was measured through FACS analysis. Three cell lines (stem cells, cancer cells, and somatic cells) were selected. The cells were classified into adherent and floating cell types, and UC-MSC-derived mitochondria were delivered into each cell type through centrifugation. Thereafter, FACS analysis was performed. The results are illustrated in FIG. 11.

From FIG. 11, it was found that mitochondria can be delivered into a wide variety of cells, although there is a difference in a delivery rate depending on cells.

### Example 5: Delivery of mitochondria into recipient cells

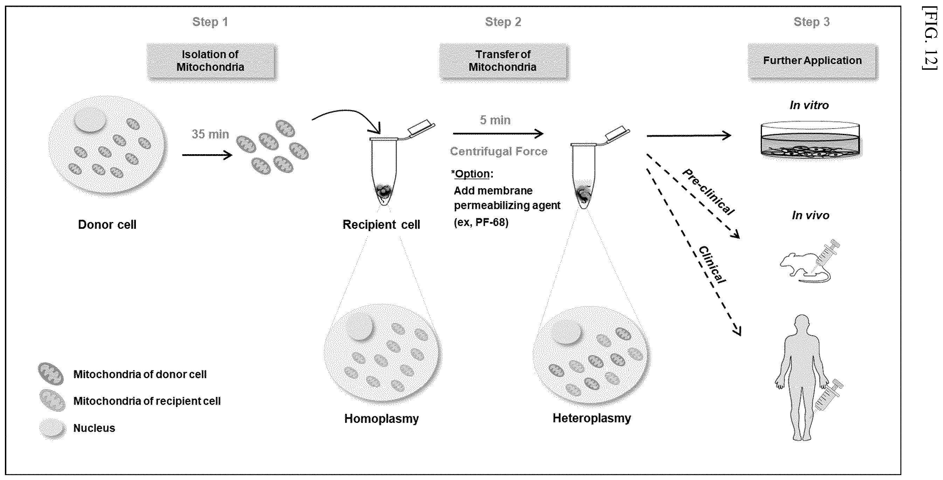

(This Example is not part of the invention.) In order to effectively deliver the exogenous mitochondria isolated according to Example 1 into recipient cells, a fast and simple centrifugation method illustrated in FIG. 12 was used. As illustrated in FIG. 12, the method can be largely divided into a process (step 1) of quickly isolating mitochondria from donor cells having a good mitochondrial activity capacity, a process (step 2) of delivering the isolated mitochondria into recipient cells, and a process (step 3) which allows the resulting cells to be applied to various applications (*in vitro, in vivo* (clinical & pre-clinical)).

### 5.1. Identification of mitochondrial delivery (1)

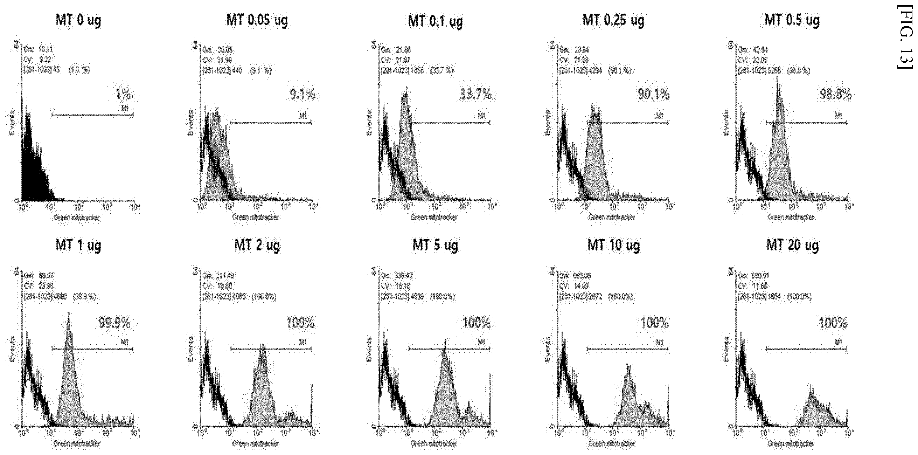

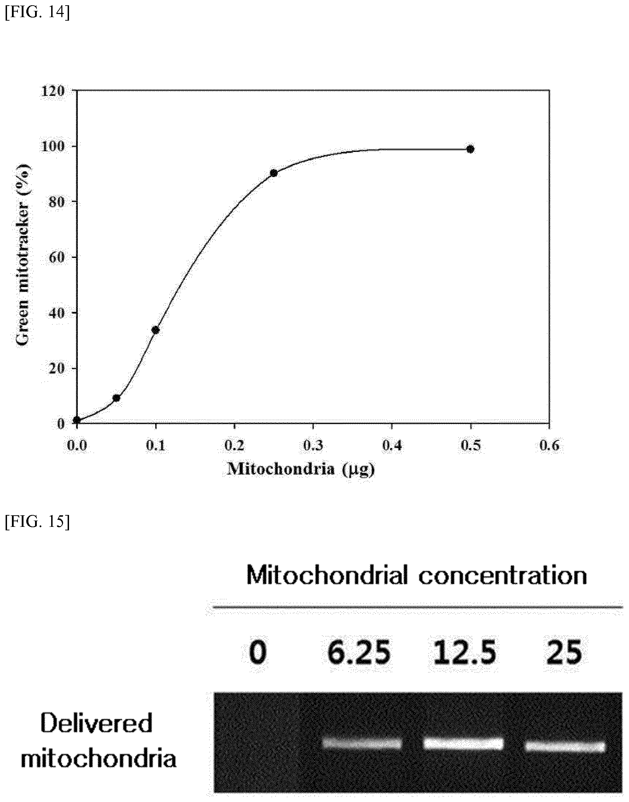

A composition obtained by mixing mitochondria extracted from WRL68 according to Example 1 with 1 × 10⁵ UC-MSCs, which are recipient cells, was treated with the method according to Example 2, so that 0.05, 0.1, 0.25, 0.5, 1, 2, 5, 10, and 20 µg of mitochondria were mixed with UC-MSCs. Here, FACS analysis was performed to measure a delivery rate depending on a concentration of mitochondria, and the results are illustrated in FIGS. 13 and 14.

From FIGS. 13 and 14, it was identified that the WRL68-derived mitochondria had been delivered into the recipient cells, UC-MSCs.

### 5.2. Identification of mitochondrial delivery (2)

Mitochondria extracted from the human liver cell line, WRL-68, and the human umbilical cord-derived mesenchymal stem cells, UC-MSCs, were mixed by the method of Example 1, and subjected to centrifugation at a temperature of about 4°C for 15 minutes with a speed of 1,500 × g. Supernatant was removed, washed with PBS, and subjected to centrifugation at a temperature of about 4°C for 5 minutes. Washing was performed twice under the same condition. In order to identify mitochondria of donor cells, which had been delivered at mitochondrial weights of 6.25, 12.5, and 25 µg per 1 × 10⁵ recipient cells, DNA in the recipient cells was extracted and identified by PCR.

A primer set (forward primer: SEQ ID NO: 1, reverse primer: SEQ ID NO: 2) of a mitochondrial gene specific for hepatocytes, WRL-68, which are donor cells, was respectively mixed with the extracted DNA, and a desired DNA portion was amplified. In order to identify a DNA product, electrophoresis on a 1.5% agarose gel was performed, and then staining with Loading Star (DYNEBIO INC., Seongnam, Korea) was performed. A UV-spectrometer (Chemi-Doc XRS; Bio-Rad Laboratories, Inc., Hercules, CA, USA) was used, and the amplified DNA bands are illustrated in FIG. 15.

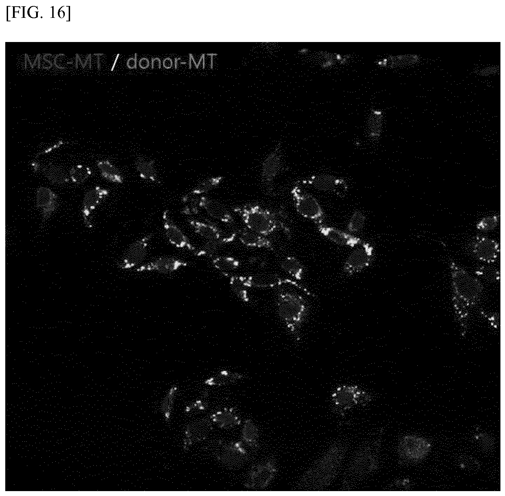

In order to identify, with a confocal scanning microscope, the mitochondria delivered into the recipient cells, the mitochondria of the donor cells and the mitochondria of the recipient cells were labeled with Mitotracker green and Mitotracker red, respectively. Twenty-four hours later, the cells were fixed on a slide using a fixative solution that contains DAPI capable of staining nuclei in blue, and intracellular mitochondria were observed using a confocal scanning microscope (Zeiss LSM 880 Confocal microscope). The results are illustrated in FIG. 16.

As illustrated in FIG. 16, it can be visually identified that the mitochondria of the donor cells have been delivered into the recipient cells.

### 5.3. Identification of optimal condition for mitochondrial delivery: Centrifugation

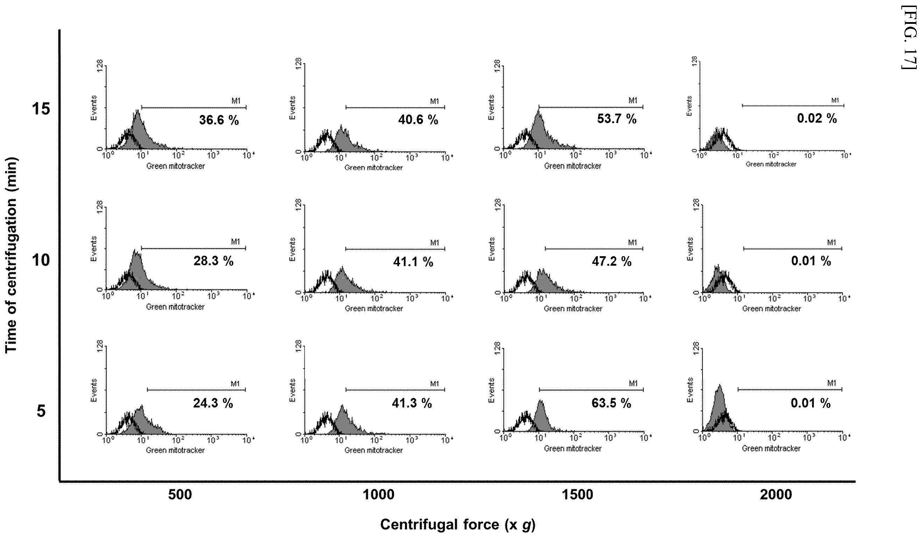

Mitochondrial delivery at respective concentrations (0.05 µg, 0.5 µg, and 5 µg) depending on various centrifugation speeds (500 × g, 1,000 × g, 1,500 × g, and 2,000 × g) and various centrifugation times (5 minutes, 10 minutes, and 15 minutes) was evaluated. The mitochondria delivered into the recipient cells depending on each condition were identified by a proportion of green fluorescence through FACS analysis. The results are illustrated in FIG. 17.

From FIG. 17, it was found that the optimal condition for delivering mitochondria into the recipient cells is a centrifugation speed of 1,500 × g and a centrifugation time of 5 minutes.

### 5.4. Identification of mitochondrial delivery according to optimal condition for mitochondrial delivery

Mitochondria isolated from the donor cells, UC-MSCs, were mixed with the recipient cells, L6 cells. Using the optimal condition for mitochondrial delivery as identified in Example 5.3, the resultant was subjected to centrifugation at a temperature of about 4°C for 5 minutes with a speed of 1,500 × g. Supernatant was removed, washing with PBS was performed twice, and centrifugation was performed at a temperature of about 4°C for 5 minutes. In order to identify mitochondria of the donor cells which had been delivered per 1 × 10⁵ recipient cells, DNA in the recipient cells was extracted and identified by PCR.

Genomic DNA (gDNA) was isolated from the recipient cells using the NucleoSpin Tissue kit (MACHEREY-NAGEL GmbH & Co. KG, Dueren, Germany), and PCR was performed under the following condition: Pre-denaturation at 95°C for 5 minutes, 25 cycles of denaturation at 95°C for 30 seconds, annealing at 58°C for 30 seconds, and extension at 72°C for 30 seconds; and final extension at 72°C for 5 minutes. In order to identify a DNA product, electrophoresis on a 1.5% (w/v) agarose gel stained with EtBr was performed, and then staining with Loading Star (DYNEBIO INC., Seongnam, Korea) was performed. A UV-spectrometer (Chemi-Doc XRS; Bio-Rad Laboratories, Inc., Hercules, CA, USA) was used to perform analysis.

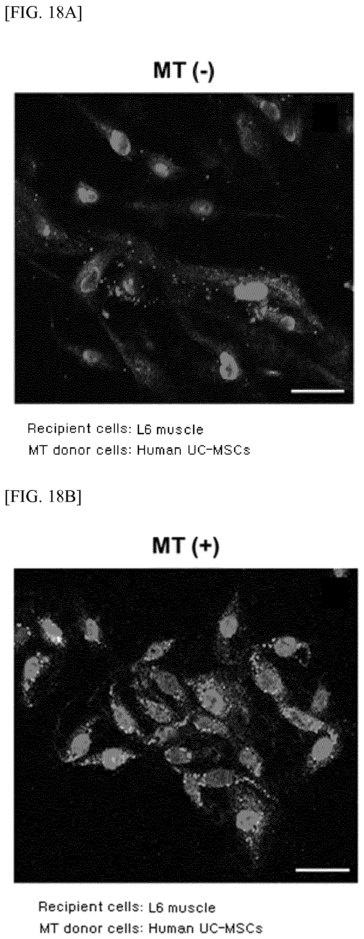

The mitochondria delivered into the recipient cells were identified by a confocal scanning microscope. Here, the mitochondria of the donor cells and the mitochondria of the recipient cells were previously stained with Mitotracker green and Mitotracker red, respectively. Then, the mitochondria underwent a mitochondria delivery process, and were observed on the basis of the recipient cells. The results are illustrated in FIGS. 18A and 18B.

As illustrated in FIGS. 18A and 18B, green fluorescence and red fluorescence were merged in the recipient cells into which the mitochondria had been delivered, resulting in a yellow mitochondrial pattern. From this, it was possible to visually identify that the mitochondria of the donor cells had been delivered into the recipient cells.

### Example 6: Measurement of mitochondrial delivery rate following treatment with surfactant

### 6.1. Measurement of delivery rate of WRL68-derived mitochondria following treatment with surfactant and incubation

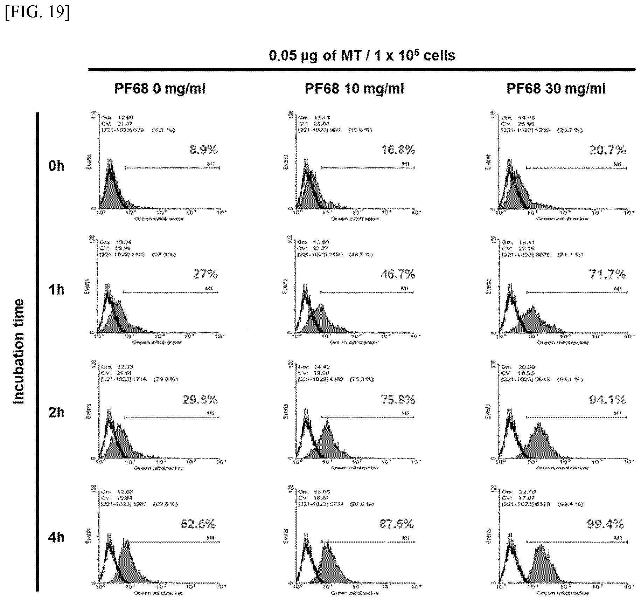

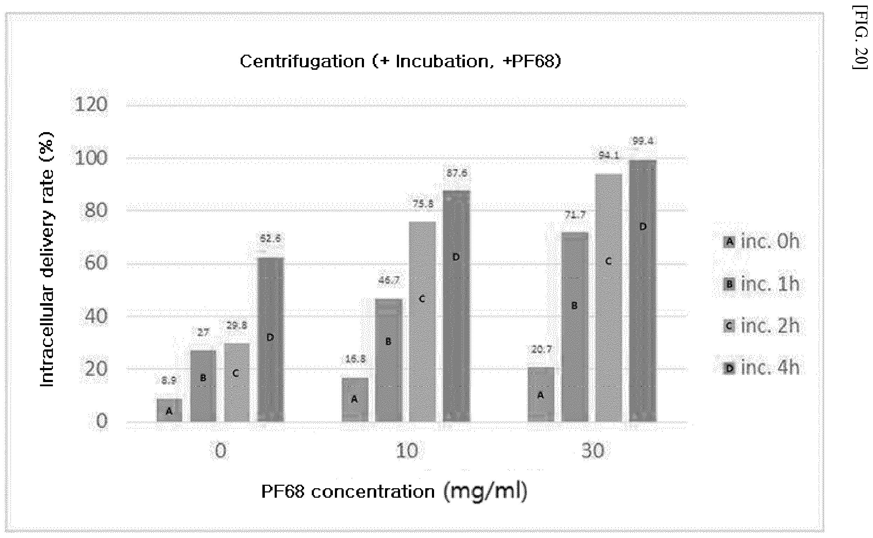

Chinese hamster ovary (CHO) cells were collected in small-sized tubes. Then, to the upper layer of the cells were added, in a small volume, liver cell line WRL68-derived mitochondria stained with Mitotracker Green. A surfactant (PF-68; BASF SE) was added to the tubes so as to be 0, 10, and 30 mg/ml, respectively, in the composition, and an incubation process was carried out at a temperature of 37°C for 0, 1, 2, and 4 hours so that a total of 12 experimental groups were constructed. Then, a centrifugation process was carried out to deliver the WRL68-derived mitochondria into the CHO cells by the method according to Example 5. This was analyzed through FACS, and the results were respectively illustrated in FIGS. 19 and 20.

As illustrated in FIGS. 19 and 20, in a case where exogenous mitochondria are introduced through incubation, it was identified that a delivery rate of the exogenous mitochondria is improved up to 99% as compared with the method (9.1% of the second histogram in FIG. 12, 8.9% of the upper left histogram in FIG. 14) in which no incubation is performed.

### 6.2. Measurement of delivery rate of UC-MSC-derived mitochondria following treatment with surfactant and incubation

Chinese hamster ovary (CHO) cells were collected in small-sized tubes. Then, to the upper layer of the cells were added, in a small volume, mitochondria of the donor cells, UC-MSCs, stained with Mitotracker Green. A surfactant (PF-68; BASF SE) was added to the tubes so as to be 0 mg/ml, 10 mg/ml, 20 mg/ml, and 30 mg/ml, respectively, in the composition, and an incubation process was carried out at 37°C for 0, 1, and 2 hours so that a total of 12 experimental groups were constructed. The results obtained by measuring, through FACS analysis, a mitochondrial delivery rate of the experimental groups for which no centrifugation process had been carried out are illustrated in FIG. 21.

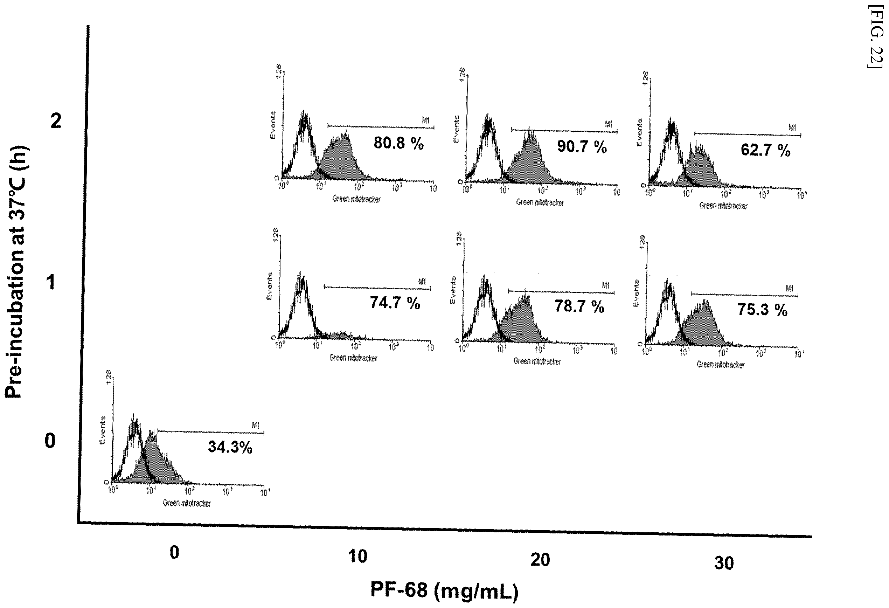

On the other hand, the results obtained by measuring, through FACS analysis, a mitochondrial delivery rate of the experimental groups, for which a centrifugation process had been carried out by the method according to Example 5 so as to deliver the mitochondria of UC-MSCs into the CHO cells, are illustrated in FIG. 22.

As illustrated in FIG. 21, a mitochondrial delivery rate of less than 10% was exhibited in the experimental group at each condition. On the other hand, in FIG. 22, a mitochondrial delivery rate of up to 90% or more was exhibited under a condition in which 20 mg/ml of PF-68 is treated for 2 hours. This means that mitochondrial delivery through centrifugation can be such that the mitochondria are delivered into the recipient cells by passing through the membrane of the cells. In addition, from the above results, it was found that treatment with a surfactant such as PF-68 regulates permeability of a cell membrane, thereby increasing a rate at which mitochondria introduced from outside can enter cells.

## Claims

1. An *ex vivo* method for delivering exogenous mitochondria into recipient cells, comprising:
a) a step of mixing the recipient cells with mitochondria isolated from donor cells; and
b) a step of centrifuging the resulting mixture;
the method further comprising a step of adding a surfactant before step b), wherein the donor cells and the recipient cells are respectively any one selected from the group consisting of somatic cells, stem cells, cancer cells, and combinations thereof.

2. The method of claim 1, wherein, in the step a), the mitochondria and the recipient cells are present in the mixture such that the mitochondria are contained in a weight of 0.005 to 25 µg per 1 × 10⁵ cells of the recipient cells.

3. The method of claim 1, wherein in the step b), the centrifugation is performed at a temperature of 0°C to 40°C for 0.5 to 20 minutes with 1 to 2,400 × g.

4. The method of claim 1, wherein the surfactant in the mixture has a concentration of 1 to 100 mg/ml.

5. The method of any one of claims 1-2, further comprising: a step of performing incubation at a temperature of 0°C to 40°C before the step b).

6. The method of claim 5, wherein the incubation is performed for 0.1 to 4 hours.

7. The method of claim 1, wherein the somatic cells are any one selected from the group consisting of muscle cells, hepatocytes, fibroblasts, epithelial cells, neurons, adipocytes, osteocytes, leukocytes, lymphocytes, mucosal cells, and combinations thereof.

8. The method of any one of claims 1-2 or 4-6, wherein the donor cells are allogeneic or xenogeneic to the recipient cells.

9. The method of any one of claims 1-2, 4-6 or 8, wherein the donor cells and the recipient cells are obtained from the same subject or from a different subject.

## Patentansprüche

1. Ex-vivo-Verfahren zum Einbringen von exogenen Mitochondrien in Empfängerzellen, umfassend:
a) einen Schritt zum Mischen der Empfängerzellen mit aus Spenderzellen isolierten Mitochondrien; und
b) einen Schritt zum Zentrifugieren des resultierenden Gemisches;
wobei das Verfahren vor Schritt b) weiter einen Schritt zum Hinzufügen eines Tensids umfasst, wobei die Spenderzellen und die Empfängerzellen jeweils solche sind, die aus der Gruppe bestehend aus somatischen Zellen, Stammzellen, Krebszellen und Kombinationen davon ausgewählt sind.

2. Verfahren nach Anspruch 1, wobei in Schritt a) die Mitochondrien und die Empfängerzellen in dem Gemisch vorhanden sind, sodass die Mitochondrien mit einem Gewicht von 0,005 bis 25 µg pro 1 × 10⁵ Zellen der Empfängerzellen enthalten sind.

3. Verfahren nach Anspruch 1, wobei in Schritt b) die Zentrifugation bei einer Temperatur von 0 °C bis 40 °C, 0,5 bis 20 Minuten lang mit 1 bis 2.400 × g durchgeführt wird.

4. Verfahren nach Anspruch 1, wobei das Tensid in dem Gemisch eine Konzentration von 1 bis 100 mg/ml aufweist.

5. Verfahren nach einem der Ansprüche 1-2, weiter umfassend: einen Schritt zum Durchführen einer Inkubation bei einer Temperatur von 0 °C bis 40 °C vor Schritt b).

6. Verfahren nach Anspruch 5, wobei die Inkubation 0,1 bis 4 Stunden lang durchgeführt wird.

7. Verfahren nach Anspruch 1, wobei die somatischen Zellen solche sind, die aus der Gruppe bestehend aus Muskelzellen, Hepatozyten, Fibroblasten, Epithelzellen, Neuronen, Adipozyten, Osteozyten, Leukozyten, Lymphozyten, Schleimhautzellen und Kombinationen davon ausgewählt sind.

8. Verfahren nach einem der Ansprüche 1-2 oder 4-6, wobei die Spenderzellen allogen oder xenogen zu den Empfängerzellen sind.

9. Verfahren nach einem der Ansprüche 1-2, 4-6 oder 8, wobei die Spenderzellen und die Empfängerzellen von demselben Subjekt oder von einem anderen Subjekt erhalten werden.

## Revendications

1. Procédé *ex vivo* d'administration de mitochondries exogènes dans des cellules réceptrices, comprenant :
a) une étape de mélange des cellules réceptrices avec des mitochondries isolées de cellules donneuses ; et
b) une étape de centrifugation du mélange résultant ;
le procédé comprenant en outre une étape d'ajout d'un tensioactif avant l'étape b), dans lequel les cellules donneuses et les cellules réceptrices sont respectivement d'un quelconque type choisi dans le groupe consistant en des cellules somatiques, des cellules souches, des cellules cancéreuses et des combinaisons de celles-ci.

2. Procédé selon la revendication 1, dans lequel, à l'étape a), les mitochondries et les cellules réceptrices sont présentes dans le mélange de telle sorte que les mitochondries sont comprises dans un poids de 0,005 à 25 µ g pour 1 × 10⁵ cellules des cellules réceptrices.

3. Procédé selon la revendication 1, dans lequel à l'étape b), la centrifugation est effectuée à une température de 0 °C à 40 °C pendant 0,5 à 20 minutes avec 1 à 2 400 × g.

4. Procédé selon la revendication 1, dans lequel le tensioactif dans le mélange présente une concentration de 1 à 100 mg/ml.

5. Procédé selon l'une quelconque des revendications 1-2, comprenant en outre : une étape de réalisation d'une incubation à une température de 0 °C à 40 °C avant l'étape b).

6. Procédé selon la revendication 5, dans lequel l'incubation est effectuée pendant 0,1 à 4 heures.

7. Procédé selon la revendication 1, dans lequel les cellules somatiques sont d'un quelconque type choisi dans le groupe consistant en des cellules musculaires, des hépatocytes, des fibroblastes, des cellules épithéliales, des neurones, des adipocytes, des ostéocytes, des leucocytes, des lymphocytes, des cellules de muqueuses et des combinaisons de ceux-ci.

8. Procédé selon l'une quelconque des revendications 1-2 ou 4-6, dans lequel les cellules donneuses sont allogéniques ou xénogéniques aux cellules réceptrices.

9. Procédé selon l'une quelconque des revendications 1-2, 4-6 ou 8, dans lequel les cellules donneuses et les cellules réceptrices sont prélevées chez un même sujet ou un sujet différent.
